# EUROPÄISCHE PATENTSCHRIFT

(11) **EP 0 931 529 B1**
(45) Veröffentlichungstag und Bekanntmachung des Hinweises auf die Patenterteilung: **14.12.2005**
(21) Anmeldenummer: 99100157.9
(22) Anmeldetag: 07.01.1999
(51) Int. Cl.: A61F 13/10

(54) **Bandage für das Handgelenk**
Wrist bandage
Bandage pour le poignet

(30) Priorität: 23.01.1998 DE 19802336
(43) Veröffentlichungstag der Anmeldung: 28.07.1999
(73) Patentinhaber: Beiersdorf Aktiengesellschaft, 20245 Hamburg (DE)
(72) Erfinder: Bodenschatz, Stefan Dr., 21614 Buxtehude (DE); Stradt, Thorsten, 22453 Hamburg (DE)

(56) Entgegenhaltungen:
- EP-A- 0 775 476
- EP-A- 0 809 988
- DE-A- 19 651 912
- GB-A- 2 307 642

## Beschreibung

Die Erfindung betrifft eine Bandage für das Handgelenk zur Ruhigstellung desselben.

Orthopädischen Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigen Material, zum Beispiel aus Neopren, Gewirke, oder Geweben. Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.
Das Zusammenfügen kann durch Nähen, Kleben oder andere übliche Verfahren erfolgen. Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Verbände oder Bandagen des Handgelenks werden bei Distorsionen, Kontusionen oder Zerrungen der ulnaren und radialen Bänder eingesetzt. Aber auch bei Fissuren der Metakarpalknochen können diese den Heilungsprozeß unterstützen. Schließlich können durch eine entsprechende Ruhigstellung des Handgelenks Reizzustände der Metakarpalgelenke bis zum völligen Verschwinden verringert werden.

Aus der EP 0 775 476 ist eine beidhändig zu tragende Bandage für das Handgelenk bekannt, die ein anatomisch gerecht geformtes, flexibles Trägermaterial einsetzt, auf das zwei Taschen jeweils im seitlichen Kantenbereich aufgenäht sind, die zur Aufnahme einer Schiene dienen. Mit Hilfe mehrerer Zügel wird die Bandage um das Handgelenk angelegt und fixiert.
Vorteilhaft an der offenbarten Bandage ist, daß diese beidhändig zu tragen ist. Es muß dazu lediglich die Schiene aus der einen Tasche entnommen und in die andere eingeschoben werden.
Nachteilig an der Bandage ist aber, daß diese zumindest einmal mittig geteilt ist, so daß die Bandage aus mehreren Stücken zusammengenäht werden muß, um eine gute Paßform am Handgelenk sicher zu stellen.
Die vorgesehenen Taschen sind dabei gerade auf das Trägermaterial aufgenäht, was beim Anlegen der Bandage zu einem unschönen und unerwünschten Faltenwurf führt.

In EP 275613 A1 wird eine Handgelenkbandage offenbart, die aus einem anatomisch geformten, das Handgelenk und die Hand aufnehmenden Abschnitt besteht, der aus einem einstückigen flexiblen und elastischen Material geformt ist. Der Abschnitt weist an der medialen Kante einen Ausschnitt zur Aufnahme des Daumens auf und an der medialen Kante sind zumindest zwei Zügel befestigt, die auf der dorsalen Seite der Bandage befestigt werden können. Weiterhin ist auf der palmaren Seite der Bandage eine aufgenähte Tasche befestigt, die gerade auf das Trägermaterial aufgenäht ist, was beim Anlegen der Bandage zu einem unschönen und unerwünschten Faltenwurf führt.

Aufgabe der Erfindung war es, eine Bandage zur Verfügung zu stellen, die durch eine explizite Paßform eine sichere und stabile Fixation des Handgelenks bei geringem Herstellungsaufwand gewährleistet und die geschilderten Nachteile des Stands der Technik nicht aufweist.

Gelöst wird diese Aufgabe durch eine Bandage, wie sie in Anspruch 1 dargelegt ist. Gegenstand der Unteransprüche sind dabei vorteilhafte Weiterbildungen der Bandage.

Demgemäß sieht die Erfindung eine Bandage für das Handgelenk vor, die aus einem anatomisch geformten, das Handgelenk und die Hand aufnehmenden Abschnitt besteht, der aus einem einstückigen flexiblen und elastischen Material geformt ist, wobei
- sich der Abschnitt in proximaler Richtung verjüngt,
- der Abschnitt an der medialen Kante einen Ausschnitt zur Aufnahme des Daumens aufweist,
- an der medialen Kante zumindest zwei Zügel befestigt sind, die auf der dorsalen Seite der Bandage befestigt werden können und
- auf der palmaren Seite der Bandage eine Tasche befestigt ist, insbesondere angenäht, dass die Mittelachse der Tasche einen einwärts, zur medialen Kante gerichteten Bogen oder Knick bildet, der einen Winkel von 150° bis 170° einschließt.

Die Tasche befindet sich dabei zumindest im Bereich des Ausschnitts zur Aufnahme des Daumens bevorzugt im Kantenbereich des Abschnitts.

Vorzugsweise sind die Zügel mit einem bekannten Klettverschluß versehen, die auf einer aufgerauhten Fläche befestigt werden, wobei die aufgerauhte Fläche auf der dorsalen Seite der Bandage derartig befestigt ist, daß die Mittelachse der Fläche in einem einwärts gerichteten Bogen plaziert ist oder die Mittelachse der Fläche zumindest einen einwärts gerichteten Knick aufweist.
Auch hier hat es sich als besonders vorteilhaft herausgestellt, wenn die Mittelachse nur einen Knick aufweist, der einen Winkel von 150° bis 170° einschließt.
Die aufgerauhte Fläche schließt sich vorzugsweise direkt an die äußere Kante des Abschnitts an.

Weiterhin vorteilhaft ist, wenn ein Zügel der Bandage am distalen Ende der medialen Kante befestigt ist und der Zügel auf der palmaren Seite mit einem Polster versehen ist.

Die Bandage besteht insbesondere aus einem in medialer und lateraler Richtung flexiblem und aus einem in proximaler und distaler Richtung weitgehend inflexiblem Material.

Daneben haben sich aber auch Neopren und vergleichbare Materialien als geeignet erwiesen.

In die Tasche der Bandage kann eine weitgehend inflexible, der Anatomie der Handinnenfläche angepaßte Schiene eingeschoben sein. Beispielsweise kann die Schiene aus Aluminium gefertigt werden.

In einer weiteren bevorzugten Ausführungsform der Bandage weist die auf der palmaren Seite der Bandage befestigte, insbesondere aufgenähte Tasche eine aufgerauhte Fläche auf, und ist die auf der dorsalen Seite der Bandage befestigte, insbesondere aufgenähte, aufgerauhte Fläche als Tasche ausgeführt. Somit sind an der Bandage zwei vorzugsweise identische Kombinationen aus Tasche/aufgerauhte Fläche vorhanden. Dadurch ist es mit nur einer einzigen Ausführungsform möglich, die Bandage an beiden Handgelenken anzulegen, sofern die Schiene in die entsprechende Tasche geschoben wird.

Die erfindungsgemäße Bandage dient als eine posttraumatische und postoperative Spezialbandage für Verletzungen im Handgelenkbereich.

Eine solche Spezialbandage muß eine explizite Paßform aufweisen, um eine sichere und stabile Fixation des Handgelenks zu gewährleisten.
Des weiteren sollte eine erfindungsgemäße Bandage einfach und kostengünstig herzustellen sein, und nicht -wie aus dem Stand der Technik bekannt- zur Erreichung der notwendigen Paßform aus vielen zugeschnittenen Teilen zusammengefügt werden müssen.
Überraschend zeigte sich darüber hinaus, daß dadurch, daß die die Schiene aufnehmende Tasche beziehungsweise die aufgerauhte Fläche nicht, wie bisher üblich, gerade auf das Trägermaterial aufgenäht werden, ein hervorragender Sitz der Bandage über dem Handgelenk ohne Faltenwurf möglich ist.

Im folgenden soll eine besonders vorteilhafte Ausführung der erfindungsgemäßen Bandage mittels mehrerer Figuren beschrieben werden, ohne damit die Erfindung unnötig einschränken zu wollen.

Im einzelnen zeigen
- die Figur 1: die erfindungsgemäße Bandage, und zwar in der Ausführung für das rechte Handgelenk, und
- die Figur 2: die Schiene der Bandage, die in die Tasche der Bandage eingeführt wird, und zwar in der Draufsicht und in seitlicher Ansicht.

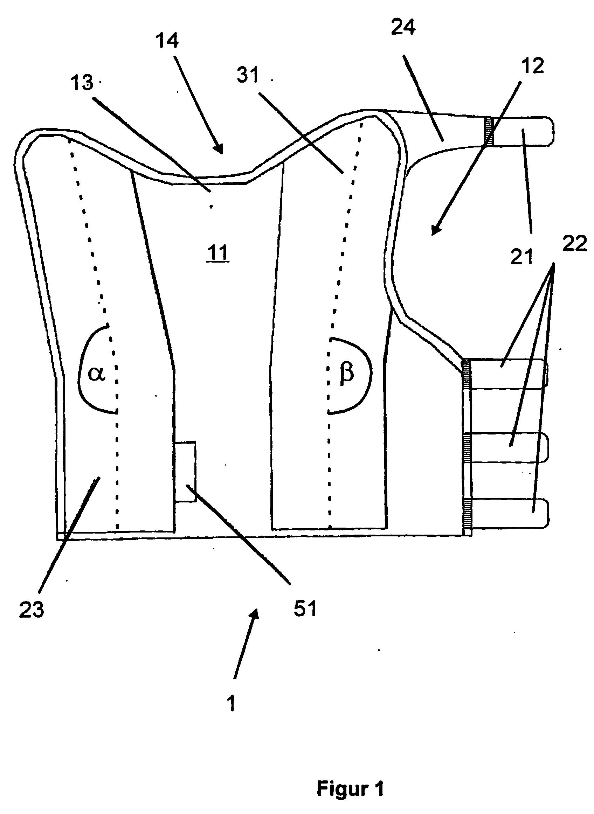

In der Figur 1 ist die erfindungsgemäße Bandage (1) in einer bevorzugten Ausführungsform im nichtangelegten Zustand gezeigt. Die Bandage (1) ist für das rechte Handgelenk bestimmt, diejenige für das linke Handgelenk besitzt einen spiegelbildlichen Aufbau.

Die Bandage (1) weist einen zentralen, anatomisch geformten, das Handgelenk und die Hand aufnehmenden Abschnitt (11) auf, der aus einem einstückigen querelastischen Material geformt ist. Der Abschnitt (11) verjüngt sich in proximaler Richtung.

Weiterhin ist an der medialen Kante ein Ausschnitt (12) vorhanden, der bei der angelegten Bandage (1) zur Aufnahme des rechten Daumens dient.
Am distalen Ende des Abschnitts (11) ist ein weiterer Ausschnitt (14) vorhanden, um die Bewegungsfähigkeit der Finger zu erhöhen.
Der Abschnitt (11) ist an allen Kanten mit einem elastischen Band (13) umnäht.

Weiterhin sind an der medialen Kante des Abschnitts (11) insgesamt vier Zügel (21, 22) befestigt, die die Fixierung der Bandage (1) an der Hand beziehungsweise an dem Handgelenk sicherstellen. Dazu weisen die Zügel (21, 22) einen Klettverschluß auf, mittels dem diese auf einer aufgerauhten Fläche (23) befestigt werden, wobei die aufgerauhte Fläche (23) auf der dorsalen Seite der Bandage (1) derartig aufgenäht ist, daß die Mittelachse der Fläche (23) einen einwärts gerichteten Knick aufweist, der einen Winkel α von 165° einschließt, wobei der Winkel an einer der äußeren Kanten der Fläche (23) eingezeichnet ist.
Hier nicht dargestellt, ist unterhalb des Zugels (21), der sich am distalen Ende der medialen Kante befindet, ein Polster (24) angebracht, das einen bequemeren Sitz der Bandage (1) ermöglicht.
Auf der palmaren Seite der Bandage (1) ist eine Tasche (31) derartig aufgenäht, daß die Mittelachse der Tasche (31) ebenso wie die Mittelachse der Fläche (23) einen Winkel β von 165° einschließt, wobei auch hier der Winkel an einer der äußeren Kanten der Tasche (31) eingezeichnet ist.
In die Tasche (31) wird eine bevorzugt aus Aluminium gefertigte Schiene (41) eingeschoben, so daß bei angelegter Bandage (1) die Fixierung und Stablisierung des Handgelenks gewährleistet ist.

Schließlich ist an der Fläche (23) ein Label (51) angenäht, auf dem angegeben werden kann, an welcher Hand die Bandage (1) anzulegen ist.

Zum Anlegen der Bandage (1) wird diese mit der gemäß Figur 1 unteren Seite auf die Handinnenfläche des Patienten derartig aufgelegt, daß der Ausschnitt (12) den Daumen aufnimmt. Die Tasche (31) mit der Schiene (41) erstreckt sich von der Handinnenfläche über das Handgelenk bis zum Unterarm. Anschließend wird der Abschnitt (11) um die Hand gelegt und mittels der Zügel (21, 22) fest fixiert.

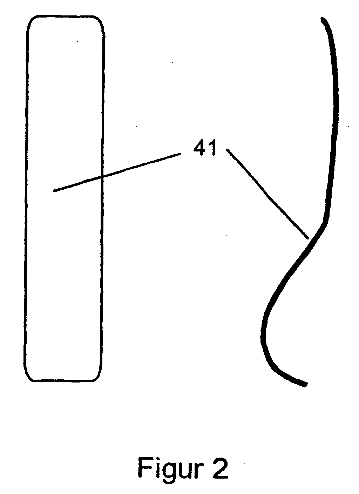

Die Figur 2 zeigt schließlich die Schiene (41) in der Draufsicht und in einer seitlichen Ansicht, die die für die Handinnenfläche anatomisch gerechte Ausformung der Schiene (41) darstellt, wobei die Schiene (41) aus Aluminium gefertigt ist, und somit weitgehend inflexibel ist.

## Patentansprüche

1. Bandage für das Handgelenk, bestehend aus einem anatomisch geformten, das Handgelenk und die Hand aufnehmenden Abschnitt, der aus einem einstückigen flexiblen und elastischen Material geformt ist, wobei
der Abschnitt an der medialen Kante einen Ausschnitt zur Aufnahme des Daumens aufweist,
an der medialen Kante zumindest zwei Zügel befestigt sind, die auf der dorsalen Seite der Bandage befestigt werden können und
auf der palmaren Seite der Bandage eine Tasche befestigt ist, **dadurch gekennzeichnet, dass**
sich der Abschnitt in proximaler Richtung verjüngt und
die Mittelachse der Tasche einen einwärts, zur medialen Kante gerichteten Bogen oder Knick bildet, der einen Winkel von 150° bis 170° einschließt.

2. Bandage nach den Anspruch 1, **dadurch gekennzeichnet, dass** die Zügel mit einem Klettverschluß versehen sind, die auf einer aufgerauhten Fläche befestigt werden, wobei die aufgerauhte Fläche auf der dorsalen Seite der Bandage derartig befestigt ist, dass die Mittelachse der Fläche in einem einwärts gerichteten Bogen plaziert ist oder die Mittelachse der Fläche zumindest einen einwärts gerichteten Knick aufweist.

3. Bandage nach Anspruch 1 oder 2, **dadurch gekennzeichnet, dass** ein Zügel der Bandage am distalen Ende der medialen Kante befestigt ist und der Zügel auf der palmaren Seite mit einem Polster versehen ist.

4. Bandage nach den Ansprüchen 1 oder 2, **dadurch gekennzeichnet, dass** die Bandage aus einem in medialer und lateraler Richtung flexiblem und aus einem in proximaler und distaler Richtung weitgehend inflexiblem Material geformt ist.

5. Bandage nach den Ansprüchen 1 oder 2, **dadurch gekennzeichnet, dass** in die Tasche der Bandage eine weitgehend inflexible, der Anatomie der Handinnenfläche angepaßte Schiene eingeschoben ist.

6. Bandage nach den Ansprüchen 1 oder 2, **dadurch gekennzeichnet, dass** die Tasche der Bandage aufgenäht ist.

7. Bandage nach mindestens einem der vorhergehenden Ansprüchen, **dadurch gekennzeichnet, dass** die auf der palmaren Seite der Bandage befestigte, insbesondere aufgenähte Tasche, eine aufgerauhte Fläche aufweist und dass die auf der dorsalen Seite der Bandage befestigte, insbesondere aufgenähte, aufgerauhte Fläche als Tasche ausgeführt ist.

## Claims

1. Wrist bandage, consisting of an anatomically shaped section which receives the wrist and the hand and which is formed from a single piece of flexible and elastic material, the section having, on the medial edge, a cutout for receiving the thumb, and
with at least two straps secured on the medial edge, which straps can be secured on the dorsal side of the bandage, and with a pocket which is secured on the palmar side of the bandage **characterized in that**, the section narrows in the proximal direction, and the centre axis of the pocket forms an inwardly directed arch towards the medial edge or forms a kink which encloses an angle of 150° to 170°.

2. Bandage according to Claim 1, **characterized in that** the straps are provided with a velcro closure which is secured on a roughened surface, the roughened surface being secured on the dorsal side of the bandage in such a way that the centre axis of the surface is located in an inwardly directed arch or the centre axis of the surface has at least one inwardly directed kink.

3. Bandage according to Claim 1 or 2, **characterized in that** one strap of the bandage is secured at the distal end of the medial edge, and the strap on the palmar side is provided with a padding.

4. Bandage according to Claim 1 or 2, **characterized in that** the bandage is made of a material which is flexible in the medial and lateral direction and substantially inflexible in the proximal and distal direction.

5. Bandage according to Claim 1 or 2, **characterized in that** a substantially inflexible splint adapted to the anatomy of the inner surface of the hand is inserted into the pocket of the bandage.

6. Bandage according to Claim 1 or 2, **characterized in that** the pocket is sewn on the bandage.

7. Bandage according to at least one of the preceding claims, **characterized in that** the pocket secured on the palmar side of the bandage, in particular sewn thereon, has a roughened surface, and **in that** the roughened surface secured on the dorsal side of the bandage, in particular sewn thereon, is designed as a pocket.

## Revendications

1. Bandage pour le poignet, se composant d'une portion de forme anatomique, recevant le poignet et la main, qui est formée d'un matériau flexible élastique d'une seule pièce, la portion présentant sur l'arête médiale une partie découpée pour recevoir le pouce, au moins deux brides étant fixées sur l'arête médiale, lesquelles peuvent être fixées sur le côté dorsal du bandage, et une poche étant fixée sur le côté palmaire du bandage, **caractérisé en ce que** la portion se rétrécit dans la direction proximale, et l'axe médian de la poche forme une courbe ou un coude orienté vers l'intérieur vers l'arête médiale, qui inclut un angle de 150° à 170°.

2. Bandage selon la revendication 1, **caractérisé en ce que** les brides sont pourvues d'une fermeture de type velcro fixée sur une surface rugueuse, la surface rugueuse étant fixée sur le côté dorsal du bandage de telle sorte que l'axe médian de la surface soit placé dans une courbe orientée vers l'intérieur ou que l'axe médian de la surface présente au moins un coude orienté vers l'intérieur.

3. Bandage selon la revendication 1 ou 2, **caractérisé en ce qu'**une bride du bandage est fixée à l'extrémité distale de l'arête médiale et la bride est pourvue du côté palmaire d'un rembourrage.

4. Bandage selon la revendication 1 ou 2, **caractérisé en ce que** le bandage est formé d'un matériau flexible dans la direction médiale et latérale et essentiellement inflexible dans la direction proximale et distale.

5. Bandage selon la revendication 1 ou 2, **caractérisé en ce qu'**un rail essentiellement inflexible, adapté à l'anatomie de la surface interne de la main, est enfoncé dans la poche du bandage.

6. Bandage selon la revendication 1 ou 2, **caractérisé en ce que** la poche du bandage est cousue.

7. Bandage selon au moins l'une quelconque des revendications précédentes, **caractérisé en ce que** la poche fixée sur le côté palmaire du bandage, notamment cousue, présente une surface rugueuse et **en ce que** la surface rugueuse fixée sur le côté dorsal du bandage, notamment cousue, est réalisée sous forme de poche.
